# EUROPEAN PATENT APPLICATION

(11) **EP 2 692 381 A1**
(43) Date of publication of application: **05.02.2014**
(21) Application number: 13179158.4
(22) Date of filing: 02.08.2013
(51) Int. Cl.: A61M 5/32

(54) **Blunt tip cannula for injection of a material into a patient**

(30) Priority: 03.08.2012 US 201261679208 P; 31.07.2013 US 201313955157
(71) Applicant: IPSYRNG Capital Development LLC, Denver, CO 80222 (US)
(72) Inventor: The designation of the inventor has not yet been filed
(74) Representative: Mallalieu, Catherine Louise

(57) **Abstract**

Cannulas that are particularly configured for the injection of a fluid such as a dermal filler into the deep/sub-dermal tissue of a patient. The cannulas may include a substantially transparent cannula wall to facilitate visibility of fluids, including aspirated blood, within the cannula. The cannulas may also include palpation elements to facilitate palpation of the cannula when the cannula is inserted into the deep/sub-dermal tissue of a patient.

## Description

### FIELD

The present disclosure relates the field of medical cannulas, particularly blunt tip cannulas that are configured for placement into the lower dermis and/or subcutaneous tissue of a patient, such as for the implantation of a medicament or a dermal filler.

### BACKGROUND

Medical procedures that include volume augmentation of a patient's skin with injectable dermal fillers have become increasingly popular. These procedures are typically performed by using a sharp hypodermic needle to inject the dermal fillers, often with the dermal filler being provided in a disposable syringe to be injected via a hypodermic needle supplied by the manufacturer. However, there are several problems associated with the use of hypodermic needles, including acute pain, tissue damage from the sharp beveled edge of the needle, bruising and vessel laceration, the need for multiple entry points (punctures), and the risk of necrosis.

Blunt tip cannulas configured for the injection of materials such as dermal fillers into the lower dermis and/or subcutaneous tissue (*e.g*., the hypodermis) of a patient have been suggested. Blunt tip cannulas may enable the injection of dermal fillers with reduced tissue trauma and using fewer entry points, increasing patient comfort and producing improved augmentation results. Despite these advantages, the use of a blunt tip cannula may lead to one or more complications such as vessel contusion, laceration, bruising, and compression necrosis. Compression necrosis may occur during or after the injection process, for example after blood vessel trauma occurs and pressure on the distressed vessel from hematoma, inflammation, and/or dermal implant insertion compresses the blood vessel in a limited space. Blunt tip cannula techniques have been developed that improve the safe injection of volumetric implant materials deeper than traditional intra-dermal injection. Blunt tip cannulas may be longer than the traditional ½ inch hypodermic needles provided with these implant materials from the manufacturer. More implant material volume may be placed in a single treatment setting, and placement may be deeper, while vessel distress may be masked by the thickness of overlaying tissues. At this point in the treatment process, needle aspiration is often desired to detect the presence of blood spill in the tissue.

### SUMMARY

Given the extra length and increased lumen sizes of blunt tip cannulas made of traditional materials such as steel, aspiration of blood is not practical and may not be present in sufficient volume to be viewed using traditional aspiration techniques, *e.g*., viewed within the most proximate portion of a glass or plastic transparent syringe body. As a result, given the current use of blunt cannulas for injection of dermal fillers, signs of potential compression necrosis may be more difficult to detect and injury complications may be more difficult to avoid. Further, although compression necroses may be detectable after treatment, the necrosis may be misdiagnosed as bruising of the skin. As a result, the necrosis may end up being treated with the application of cold (e.g., ice) as opposed to the desirable treatment with heat.

One objective of the embodiments disclosed herein it is to enable the early detection of complications during injection of a material into the deep/sub-dermal tissue of a patient, such as vessel contusion, lacerations, bruising and compression necrosis. Another objective is to enable the detection of fluids such as blood within the cannula. It is another objective to provide a cannula having enhanced palpation properties. Any one or more of these objectives may be met by one or more of the following embodiments.

In one embodiment, a cannula that is configured for the injection of materials into the deep/sub-dermal tissue of a mammalian patient is provided. The cannula may include a longitudinally-extending cannula wall forming a circumferentially closed cylinder, the closed cylinder having an inner diameter defining a fluid channel along a length of the closed cylinder and having an outer diameter. A blunt tip may be disposed at a distal end of the closed cylinder and a proximal fluid port may be disposed at a proximal end of the closed cylinder that is configured to provide fluid access to the fluid channel. At least a first distal fluid port may be disposed at a distal end of the closed cylinder and may be configured to provide fluid access to the fluid channel. In this embodiment, the cannula wall may be fabricated from a polymeric material.

In one characterization of this embodiment, the cannula wall is substantially transparent, *e.g*., such that fluids such as blood may be visually observed in the cannula under normal lighting conditions. In another characterization, the polymeric material from which the cannula wall is fabricated is an anisotropic polymer. In another characterization, the polymeric material is an extruded polymeric material. In yet another characterization, the polymeric material is selected from the group consisting of nylon, polypropylene, polyesters, polyamides, polyurethanes, polyvinylchloride (PVC), polyolefin and thermoplastic elastomer, polyacrylates, polycarbonates, polystyrenes, and modified or unmodified polyethylene terephthalates (PET).

In another characterization, the cannula wall may have a thickness of at least about 0.003 inches (0.076 mm). In another characterization, the cannula wall may have a thickness of not greater than about 0.20 inches (5.08 mm). In yet another characterization, the fluid channel has a diameter of at least about 0.001 (0.025 mm) inches and not greater than about 0.30 inches (7.6 mm). The cannula may have a length of at least about 0.25 inches (6.35 mm) and not greater than about 6 inches (152 mm).

In another characterization, the blunt tip is a closed blunt tip. In another characterization, the at least first distal fluid port is disposed through the blunt tip. In yet another characterization, the at least first distal fluid port is disposed through the cannula wall.

In another characterization, the cannula may include at least a first visualization port disposed through the cannula wall nearer the proximal end of the cannula than the at least first distal fluid port. In one characterization, the cannula wall is substantially rigid. In another characterization, the cannula wall is flexible. In a further characterization, the blunt tip includes a bulbous protrusion having an outer diameter that is greater than an outer diameter of the closed cylinder. The bulbous protrusion may be particularly configured for palpitation of the cannula. The bulbous protrusion may have an outer diameter that is at least about 10% greater than the outer diameter of the closed cylinder and not more than about 30% greater than the outer diameter of the closed cylinder. In one characterization, the bulbous protrusion is substantially spherical. In another characterization, the bulbous protrusion has a tapered cross-section. In yet another characterization, the bulbous pultrusion is substantially ovoid in shape. In yet another characterization, the bulbous pultrusion is integrally formed with the cannula wall.

In another characterization, at least a portion of the cannula wall is colored to facilitate visibility of the cannula wall when the cannula is at least partially inserted under the skin of a patient. In yet another characterization, a light source may be operatively connected to the cannula and configured to illuminate the cannula wall when the cannula is at least partially inserted under the skin of a patient. In one characterization, the light source is a light emitting diode (LED). In another characterization, the cannula includes at least a first longitudinally extending reinforcing ribs disposed along the length of the fluid channel.

In another embodiment disclosed herein, a method for injecting an implant material into the skin tissue of a mammalian patient is provided. The method may include the steps of forming an implantation puncture through the epidermis of the patient. A blunt cannula may be inserted into the implantation puncture, where the blunt cannula is substantially as described herein. An implant material may then be injected into the cannula.

In one characterization of this embodiment, the implant material may include dermal filler. In another characterization, the implant material may include autologous fat. In yet another characterization, the implant material may be configured for long term release of a medicament into the patient.

In another characterization, the cannula wall is substantially transparent. In this regard, the method may include the steps of aspirating blood, after the insertion step, into the fluid channel and observing, through the cannula wall, an amount of aspirated blood in the fluid channel. Further, the cannula may include a bulbous pultrusion (*e.g*., a palpating element) and the method may include the step of palpating to determine the position of the bulbous pultrusion in the deep/sub-dermal tissue of the patient.

### DESCRIPTION OF THE DRAWINGS

**Fig. 1** illustrates a perspective view of a blunt tip cannula.

**Fig. 2** illustrates a cross-sectional view of a blunt tip cannula taken along the longitudinal extent of the cannula.

**Fig. 3** illustrates a perspective view of a blunt tip cannula.

**Fig. 4** illustrates a perspective view of a blunt tip cannula.

**Fig. 5** illustrates a perspective view of a blunt tip cannula.

**Fig. 6** illustrates a cross-sectional view of a blunt tip cannula taken perpendicular to a longitudinal extent of the cannula.

**Fig. 7** illustrates a perspective view of a blunt tip cannula.

**Fig. 8** illustrates a cross-sectional view of a blunt tip cannula taken perpendicular to a longitudinal extent of the cannula.

**Fig. 9** illustrates a perspective view of a blunt tip cannula having a bulbous protrusion at a distal end thereof.

**Fig. 10** illustrates a perspective view of a blunt tip cannula having a bulbous protrusion at a distal end thereof.

**Fig. 11** illustrates a perspective view of a blunt tip cannula having a bulbous protrusion at a distal end thereof.

**Fig. 12** illustrates a perspective view of a blunt tip cannula having a bulbous protrusion at a distal end thereof.

**Fig. 13** illustrates a perspective view of a blunt tip cannula having an attachment hub operatively affixed to the cannula.

**Fig. 14** illustrates a device for the injection of a material into a patient including a blunt tip cannula.

**Fig. 15** illustrates a blunt tip cannula having a light source operatively connected to the cannula.

### DESCRIPTION

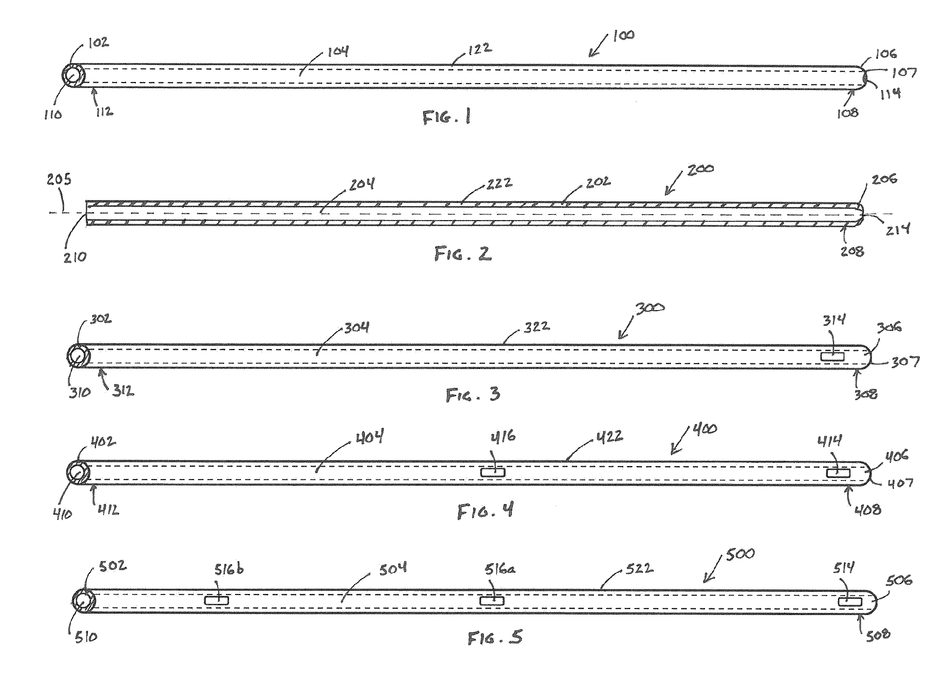

**Figs. 1-5** illustrate several embodiments of a cannula in accordance with the present disclosure. **Fig. 1** illustrates a perspective view of a blunt tip cannula **100.** The cannula **100** is particularly configured for the injection of materials (*e.g*., fillers such as dermal fillers) into tissue including, but not limited to, the lower dermis, deep dermis, subcutaneous tissue, hypodermis and/or periosteum (collectively referred to herein as the " deep/sub-dermal tissue") of a patient (*e.g*., a human or other mammalian subject). The cannula **100** includes a longitudinally-extending cannula wall **102** that forms a circumferentially closed cylinder **122.** That is, with the possible exception of certain apertures (*e.g*., ports) that may be selectively placed along the length of the cannula (discussed below), the cannula wall **102** is continuous along its circumferential extent. The closed cylinder **122** has an inner diameter defining fluid channel **104** along a length of the closed cylinder **122.** Although illustrated as a right circular cylinder, it will be appreciated that the closed cylinder may also be a non-circular cylinder, such as an elliptical cylinder, *e.g*., having an elliptical cross-section.

Disposed at a distal end **108** of the cannula **100** is a blunt tip **106.** The blunt tip **106** may include, for example, a tapered and smoothly rounded surface **107** to facilitate the insertion of the distal end **108** of the cannula **100** into the deep/sub-dermal tissue of a patient while reducing the likelihood of bruising or necrosis in the patient. That is, the blunt tip **106** is relatively dull and is configured for blunt dissection through the deep/sub-dermal tissue as opposed to the sharp cutting dissection of tissues that occurs with the use of sharp needles, such as hypodermic needles.

A proximal fluid port **110** (*e.g*., an aperture) is disposed at or near a proximal end **112** of the cannula **100,** *e.g*., at a proximal end **112** of the closed cylinder **122.** The proximal fluid port **110** is configured to provide fluid access to the fluid channel **104** and is configured such that flowable fluids (*e.g*., liquids, gels, gases) may be passed through the proximal fluid port **110** and into the fluid channel **104** (*e.g*., under light to moderate pressure). For example, the proximal fluid port **110** may be operatively attached to a syringe having a plunger for injecting a fluid into the fluid channel **104** through the proximal fluid port **110.**

At least a first distal fluid port **114** (*e.g*., an aperture) is disposed at or near (*e.g*., proximate to) the distal end **108** of the cannula **100.** The distal fluid port **114** is configured to provide fluid access to the fluid channel **104,** *e.g*., to permit ingress or egress of fluid to or from the fluid channel **104.** As a result, the proximal fluid port **110** and the distal fluid port **114** are in fluid communication via the fluid channel **104,** thereby enabling a potentially present body fluid to be aspirated into the fluid channel **104** from distal fluid port **114,** and also enabling a fluid to be injected at the proximal fluid port **110** and to be ejected from the distal fluid port **114** during injection when sufficient pressure is applied to the fluid disposed within the fluid channel **104,** *e.g*., by using a syringe having a plunger that is operatively connected to the cannula **100.** As used herein, the term "fluid" includes any flowable medium, including but not limited to suspensions, *e.g*., suspensions of solid particulates in a flowable fluid medium and body fluids including but not limited to, *e.g*., blood.

**Fig. 2** illustrates a cross-sectional view of a cannula of similar structure to the cannula of **Fig. 1****,** taken along a longitudinal extent of the cannula. The cannula **200** includes a longitudinally-extending cannula wall **202** that forms a circumferentially closed cylinder **222.** The closed cylinder **222** has an inner diameter defining a fluid channel **204.** A proximal fluid port **210** and an distal fluid port **214** are configured to provide fluid access to the fluid channel **204,** that fluidly connects the proximal fluid port **210** to the distal fluid port **214** such that the ports are in fluid communication. A blunt tip **206** is disposed at a distal end of the cannula **200.** As illustrated in **Fig. 2****,** the proximal fluid port **210** and the distal fluid port **214** intersect a longitudinal axis **205** of the fluid channel **204** (*e.g*., of the cannula **200).** For example, the distal fluid port **214** is disposed through the blunt tip **206** of the cannula **200** at the distal end **208.**

Cannulas of the type used for dermal injections are typically fabricated from a metal, *e.g*., an opaque metal. In accordance with the cannulas disclosed herein, at least the cannula wall (*e.g*., walls **102** and **202)** may advantageously be fabricated from a polymeric material, such as a plastic material. In one characterization, the entire cannula **100** is fabricated from a polymeric material. For example, the polymeric material may be an anisotropic polymer having anisotropic mechanical properties. In one characterization, the polymeric material is an extruded polymeric material, *e.g*., is produced by extrusion of the polymeric material through an extrusion die. Examples of polymeric materials from which the cannula wall may be constructed are those having a high strength (*e.g*., high tensile strength), and light weight. Examples of useful polymers may include, but are not limited to, nylon, polypropylene, polyesters, polyamides, polyimides, polyurethanes, polyvinyl chloride (PVC), polyolefins, thermoplastic elastomers, polyacrylates, polycarbonates and modified or unmodified polyethylene terephthalates (PET), liquid crystal polymers (LCPs) and the like.

The cannula wall may also be formed from two or more different polymers, *e.g*., two different polymers formed in distinct layers such as concentric layers. For example, the two different polymers may have different refractive indices and/or densities to facilitate the passage of light along the length of the cannula wall, as is described in more detail below. The two or more different polymer layers may be formed by co-extrusion of two different polymers, for example.

In one characterization, the cannula wall is configured to be substantially transparent, *e.g*., translucent, to visible light. That is, the cannula walls may be sufficiently transparent such that materials (*e.g*., medicaments, fillers and/or blood) contained within the fluid channel are readily visible through the cannula wall, *e.g*., under normal external lighting conditions. Transparency may be achieved by careful selection of the polymeric material. Examples of polymeric materials that may be useful for substantially transparent cannula walls, while maintaining adequate strength properties, may include but are not limited to, polyacrylates, polycarbonates, polystyrenes, and modified or unmodified polyethylene terephthalates (PET). To enhance transparency, the polymer may be substantially amorphous, *e.g*., containing less than 5% crystalline phase, or substantially crystalline, *e.g*., greater than 75% crystalline.

Using prior blunt cannula configurations, the operator (*e.g*., a surgeon) may inadvertently lacerate or otherwise injure a blood vessel causing the vessel to lose pressure, among other symptoms. In such incidents, vessels may become vulnerable to compression forces resulting from inflammation, bleeding, and/or the pressure of injected implant materials. The injuries resulting from compression include but are not limited to vasospasm, impending necrosis, and full compression necrosis with tissue slough, severe pain, scarring, and infection risk. The operator may be unaware that bleeding is occurring unless, during aspiration, the blood travels all the way up the entire length of the fluid channel, out of a proximal fluid port and into the syringe where it is visible. A substantially transparent cannula wall may advantageously enable the aspirated blood to be observed in the fluid channel almost immediately, allowing the operator to take corrective action in a timely manner, including not implanting filler material at the vessel injury site, therefore reducing the possibility of necrosis as well as avoiding more involved and high risk treatments to address pending or active necrosis. In addition, a transparent cannula wall may facilitate visibility of the implant filler material in the fluid channel, enabling greater control over the placement of the material by the operator, *e.g*. being able to visualize material blockage or movement of material within the cannula. These advantages may be particularly important for the placement of dermal fillers into the deep/sub-dermal tissue of a patient and to enable the operator to determine the force being put on the syringe, *e.g*., due to material blocking the fluid channel.

**Fig. 3** illustrates a perspective view of a cannula **300** having a polymeric cannula wall **302** that is substantially transparent, *e.g*., is sufficiently transparent such that materials contained within the fluid channel **304** are readily visible through the cannula wall **302.** The cannula wall **302** forms a closed cylinder **322** that has an inner diameter defining a fluid channel **304** along the length of the cylinder **322.** The distal end **308** of the cannula **300** includes a blunt tip **306** having a rounded surface **307,** *e.g*., that is devoid of sharp edges. A proximal fluid port **310** is disposed at a proximal end **312** and a distal fluid port **314** (*e.g*., an aperture) is disposed proximate to the distal end **308.** Thus, the fluid channel **304** provides fluid communication between the proximal fluid port **310** and the distal fluid port **314** so that fluid may be injected through proximal fluid port **310** and may be ejected from and body fluid may be alternately aspirated into the distal fluid port **314,** *e.g*., when the distal end **308** of the cannula **300** is inserted into the deep/sub-dermal tissue of a patient. As illustrated in **Fig. 3****,** the distal fluid port **314** is disposed through the cannula wall **302,** *e.g*., does not intersect a longitudinal axis **305** of the fluid channel **304.**

**Fig. 4** illustrates a perspective view of a cannula that includes an additional "visualization fluid port" for increased access of body fluid to be viewed during aspiration in deep/sub-dermal tissue of a patient. This additional port also serves to enable outflow of the fluid to be caused using less force to inject, enabling a smoother, more comfortable injection procedure for the operator. The cannula **400** includes a cannula wall **402** that forms a circumferentially closed cylinder **422** having an inner diameter defining a fluid channel **410** that provides fluid communication between a proximal fluid port **410** and a distal fluid port **414** that is disposed proximate a distal end **408** and proximate a blunt tip **406.** An additional visualization port **416** (*e.g*., an aperture) is disposed in the cannula wall **402** nearer the proximal end **412** of the cannula **400** than the distal fluid port **414.** In one characterization, the visualization port **416** is spaced from the blunt tip **406** (*e.g*., from the rounded surface **407** of the blunt tip **406)** toward the proximal end **412** by a distance of at least about 0.2 inches (200 mils), such as near the middle of the cannula. In another characterization, the visualization port **416** is spaced from the blunt tip **406** by a distance that is at least 30% of the total length of the cannula **400,** such as at least 40% of the total length of the cannula **400.** In another characterization, the visualization port is spaced from the blunt tip by a distance of not more than about 70% of the total length of the cannula **400,** such as not greater than about 60% of the total length of the cannula **400.**

The visualization port **416** may be utilized either with or without a cannula wall **402** that is substantially transparent. In one characterization, the cannula wall **402** is a polymeric material that is not substantially transparent, *i.e.,* is opaque. In this characterization, the visualization port **416** provides a means for detecting the aspiration of blood within the fluid channel **404** before the blood reaches the proximal fluid port **410** so that the operator of the cannula **400** may take corrective action. The visualization port **416** may extend entirely through the cannula wall **402,** *e.g*., in a manner similar to distal fluid port **414.** Alternatively, the visualization port **416** may extend only part way through the cannula wall **402** such that the fluid cannot pass through the visualization port **414.** For example, a thin polymer layer (*e.g*., thinner than the cannula wall **402)** may be disposed between the visualization port **414** and the fluid channel **404,** or over the top of the visualization port **414,** such as to enable a fluid to be visually observed through the port **414.**

**Fig. 5** illustrates a cannula **500** of similar construction to the cannula illustrated in **Fig. 4****.** The cannula wall **502** forms a closed cylinder **522** that has an inner diameter defining a fluid channel **504** along the length of the cylinder **522.** The cannula **500** includes blunt tip **506** at a distal end **508** and a distal fluid port **514** that is in fluid communication with a proximal fluid port **510** disposed at a proximate end **412** of the cannula through a fluid channel **504.** As illustrated in **Fig. 5****,** the cannula **500** includes a first visualization port **516a** and a second visualization port **516b** in the cannula wall **502.** As with the embodiment illustrated in **Fig. 4****,** the cannula wall **502** may or may not be substantially transparent, and in one characterization the cannula wall **502** is opaque, and the visualization ports **516a/516b** may be configured in a manner similar to visualization port **416.**

To facilitate use of the cannula (*e.g*., the cannulas illustrated in **Figs. 1-5****)** for the injection of fluids into the deep/sub-dermal tissue of a patient, the cannula may have a total length of at least about 0.25 inches (6.35 mm) and not greater than about 8.0 inches (203 mm), such as not greater than about 6.0 inches (152 mm) for example. It will be appreciated that the length of the cannula may be selected for a particular application with the goal of reducing puncture/entry wound sites.

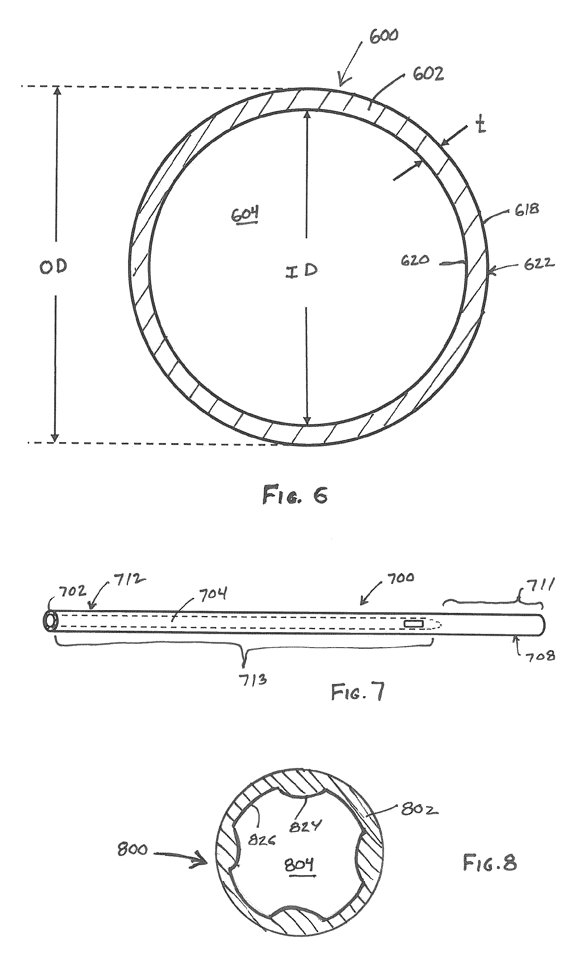

**Fig. 6** illustrates a cross-sectional view of a cannula **600** that is taken perpendicular (*e.g*., transverse) to a longitudinal axis of the cannula **600** (*e.g*., see **Fig. 2****).** The cannula **600** includes a cannula wall **602** that forms a closed cylinder **622.** The closed cylinder **622** has an inner wall surface **620** and an outer wall surface **618,** where the inner wall surface **620** defines a fluid channel **604** through the cannula **600.** The following description of the cannula **600** may apply equally to the cannulas illustrated in **Figs. 1-5** above or the cannulas illustrated in **Figs. 7-12** below, for example.

The cannula wall **602** should have a sufficient thickness such that the likelihood of accidentally puncturing, breaking, or tearing the cannula wall **602** is very low, and so that the cannula **600** maintains sufficient rigidity so that it may be manipulated and inserted into the deep/sub-dermal tissue of a patient. In one characterization, the cannula wall **602** has a thickness **(t)** of at least about 0.003 inches (0.076 mm) such as at least about 0.01 inches (0.254 mm). However, the wall thickness must be selected to provide both a sufficient inner diameter **(ID)** to facilitate the passage of a material through the fluid channel **604,** while maintaining the outer diameter **(OD)** sufficiently small that the cannula **600** may be inserted into the deep/sub-dermal tissue of a patient without causing significant discomfort and with a goal of requiring the smallest possible insertion wound. In those embodiments where the cannula wall **602** is substantially transparent to facilitate visibility of fluid in the fluid channel **604,** the cannula wall **602** should be sufficiently thin to facilitate such transparency. In one characterization, the cannula wall **602** has a thickness of not greater than about 0.04 inches (1.0 mm), such as not greater than about 0.02 inches (0.5 mm).

As is noted above, the inner diameter "**ID**" of the fluid channel **604** (*i.e.,* the diameter of the inner wall surface **620)** should be sufficient to enable the flow of a fluid, such as dermal filler or body fluid, through the fluid channel **604.** In one characterization, the minimum diameter of the fluid channel **604** may be at least about 0.020 inches (0.5 mm), such as at least about 0.040 inches (1.0 mm). In another characterization, the maximum inner diameter of the fluid channel **604** may be not greater than about 0.158 inches (4.0 mm) such as not greater than about 0.118 inches (3.0 mm). In the event that the fluid channel **604** is not substantially circular (*e.g*., is elliptical), the minimum diameter may be taken along a minor axis and the maximum diameter may be taken along a major axis of the ellipse. In one characterization, an elliptical cross-section may advantageously increase the rigidity of the cannula at the same wall thickness as an otherwise identical cannula having a circular cross-section.

Also, the outer diameter **"OD"** of the cannula wall **602** (*i.e.,* the outer diameter of the outer wall surface **618)** may be not greater than about 0.236 inches, (6.0 mm) such as not greater than about 0.157 inches (4.0 mm). In another characterization, the outer diameter is at least about 0.026 inches (0.66 mm), such as at least about 0.06 inches (1.5 mm). Further, the outer diameter of the cannula wall **602** may be coated or otherwise treated to reduce the friction experienced by the cannula wall **602.** In one characterization, the cannula wall **602** is coated with a fluoropolymer.

Although the cannulas of **Figs. 1-6** are illustrated as having a constant wall thickness **t** along the length of the cannula wall, the wall thickness may vary along the length of the cannula wall, *e.g*., where the outer diameter of the cannula wall changes along its length and/or where the inner diameter ID of the fluid channel changes along a length of the fluid channel. Such a configuration may advantageously vary the rigidity of the cannula from the distal end to the proximal end. In one characterization, the cannula wall thickness is greater near the distal end of the cannula and is lower near the proximal end to provide rigidity for insertion of the distal end into the deep/sub-dermal tissue and greater flexibility of the cannula near the proximal end. For example, the diameter of the fluid channel may increase from the distal end toward the proximal end in a substantially constant or a step-wise fashion.

For example, **Fig. 7** illustrates a cannula **700** wherein an inner diameter of the cannula wall **702** (*e.g*., the diameter of the fluid channel **704)** varies along a length of the cannula **700,** particularly where the fluid channel **704** tapers toward a distal end **708** and terminates before the distal end **708.** In this manner, the cannula **700** may include a rigid portion **711** adjacent the distal end and a more flexible portion **713** near the proximal end **712.** Such a configuration may advantageously provide a high stiffness at the distal end for insertion of the cannula, and a higher flexibility near the proximate end of the cannula for greater flexibility and maneuverability of the cannula after insertion.

**Fig. 8** illustrates a cross-sectional view (*e.g*., taken perpendicular to a longitudinal axis) of a cannula **800** having a profiled inner diameter, e.g., of the fluid channel. The cannula **800** includes a cannula wall **802** having a plurality of reinforcing ribs **824** that run along (*e.g*., longitudinally extend along) a length of the cannula wall **802** (*e.g*., along a length of the fluid channel **804).** Characterized another way, the cannula wall **802** has a first thickness along the reinforcing ribs **824** that is greater than a second thickness of gap portions **826** that are disposed between adjacent reinforcing ribs **824.** In this manner, the reinforcing ribs **824** may provide stiffness to the cannula **800** while the gap portions **826** provide enhanced transparency due to the thinner wall along the gap portions **826.** Although illustrated as comprising four reinforcing ribs **824,** the cannula may include a single reinforcing rib or any number of reinforcing ribs **824.** The size (*e.g*., the thickness) of the reinforcing ribs **824** can also be varied to increase or decrease the stiffness of the cannula **800.**

In another characterization, reinforcing ribs may be disposed substantially perpendicular to the longitudinal axis of the cannula (*e.g*., circumventing the fluid channel), in addition to or in lieu of the longitudinally extending ribs, such as to enable curve of the cannula along a specified arc, *e.g*., to substantially conform to a particular facial feature. In this regard, although the cannulas are illustrated as being substantially linear (*e.g*., straight along their length), the cannulas may be curved along a length of the cannula, such as a along the entire length of the cannula or a portion of the entire length.

In a further embodiment, the cannulas disclosed herein may comprise an element that facilitates the palpation of the blunt tip of the cannula when the cannula is inserted into the deep/sub-dermal tissue of a patient. That is, blunt tip cannulas are often used to precisely place materials such as dermal fillers in the deep/sub-dermal tissue of a patient. However, existing cannula designs do not enable the cannula to be easily palpated by an operator (*e.g*., a surgeon), and therefore do not enable the precise location of the blunt tip within the skin tissue to be easily determined.

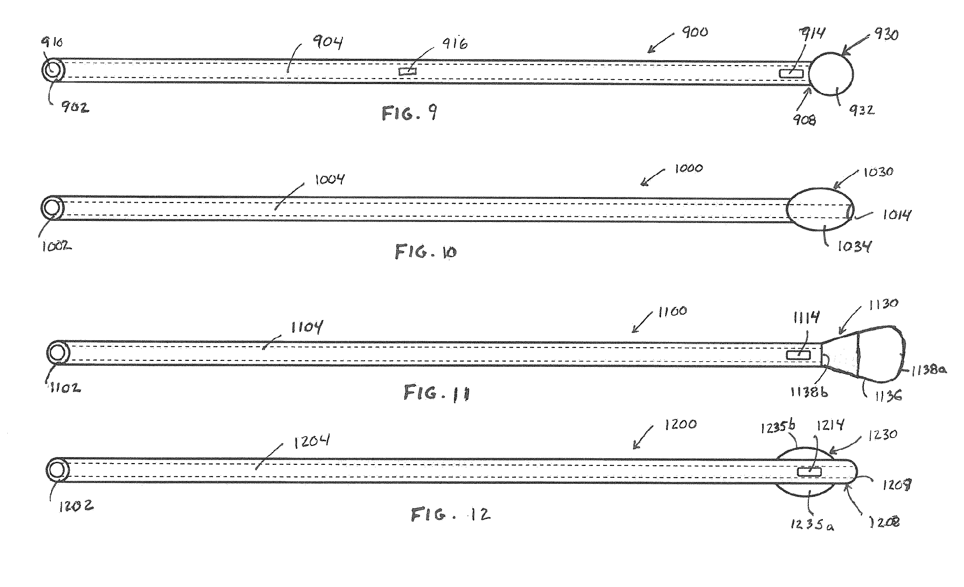

**Fig. 9** illustrates a cannula that includes such a palpation element. The cannula **900** may or may not be fabricated from a polymeric material (*e.g*., a substantially transparent polymeric material) as is discussed above, and may or may not include a visualization port **916** as illustrated in **Fig. 9****.** The cannula **900** includes a proximal fluid port **910** and a distal fluid port **914** that are in fluid communication through a fluid channel **904.** The cannula **900** also includes a bulbous protrusion **930** to enable palpation of the distal end **908** of the cannula **900.** As illustrated in **Fig. 9****,** the bulbous protrusion **930** is in the form of a sphere **932.** The sphere **932** has an outer diameter that is greater than the outer diameter of the cannula wall **902** so that the sphere **932** may be readily palpated after the insertion of the distal end **908** into the deep/sub-dermal tissue of a patient. Although the distal fluid port **914** is illustrated as being disposed in the cannula wall **902,** the distal fluid port may be disposed within the bulbous protrusion **930,** *e.g*., where the fluid channel **904** extends into the bulbous protrusion **930,** *e.g*., extends through the bulbous protrusion **930** and provides a fluid connection to the fluid channel **904.**

**Figs 10-12** illustrate alternative embodiments of a cannula having a bulbous protrusion to facilitate palpation of the distal end of the cannula. In the embodiment illustrated in **Fig. 10****,** the cannula **1000** includes a bulbous protrusion **1030** in the form of a prolate (elongated) spheroid **1034.** The prolate spheroid **1034** has an outer diameter that is greater than the outer diameter of the cannula wall **1002** to facilitate palpation. As illustrated in **Fig. 10****,** the fluid channel **1004** extends through the bulbous protrusion **1030** to a distal fluid port **1014** disposed at the end tip of the bulbous protrusion **1030.** Although illustrated as comprising a prolate spheroid **1034,** the bulbous protrusion may also comprise an oblate (flattened) spheroid, or other similar geometric configurations.

**Fig. 11** illustrates a cannula **1100** that includes a bulbous protrusion **1130** comprising the general shape of a frustum **1136** having a rounded (*e.g*., smooth) distal base **1138a.** The diameter of the frustum **1136** at the distal base **1138a** is greater than the diameter of the proximal base **1138b** of the frustum **1136.** Although illustrated as a frustum **1136,** the bulbous protrusion could also comprise a cylinder, *e.g*., a cylinder having an outer diameter that is greater than the outer diameter of the cannula wall **1102.**

**Fig. 12** illustrates a cannula **1200** having a bulbous protrusion **1230** that is spaced away from the distal tip **1209** of the cannula **1200.** The bulbous protrusion **1230** comprises fins **1235a** and **1235b** that are disposed on opposite sides of the cannula wall **1002** proximate the distal end **1208** of the cannula **1200.** A distal fluid port **1214** is disposed between the fins **1035a** and **1035b** and provides fluid access to the fluid channel **1204.** It will be appreciated that the bulbous protrusion may comprise a single fin, or more than two fins, and in one characterization comprises at least three fins disposed on the cannula wall **1202.** Further, although the fins are illustrated as being in the form of an arc, other shapes are contemplated for use as a bulbous protrusion.

To facilitate palpation of the distal end of the cannulas, the bulbous protrusions may have an outer diameter that is at least about 10% greater than the outer diameter of the cannula wall, such as at least about 15% greater that the outer diameter of the cannula wall. The outer diameter of the bulbous protrusion may be taken as the outer diameter of a circle that would encompass the bulbous protrusion at its largest cross-section (*e.g*., transverse to the longitudinal axis of the closed cylinder formed by the cannula wall). In another characterization, the outer diameter may be not greater than about 30% of the outer diameter of the cannula wall. In yet a further characterization, the outer diameter of the bulbous protrusion is at least about 0.14 inches (3.6 mm), and is not greater than about 0.24 inches (5.9 mm).

The bulbous protrusion(s) may be integrally formed with the cannula wall, *e.g*., may comprise the same material as the cannula wall such as a polymer, including a substantially transparent polymer. Such an integrated bulbous protrusion may be formed, for example, by micro-molding or micro-machining. Integrally forming the bulbous protrusion with the cannula wall may reduce or eliminate the chance that the bulbous protrusion will separate from the cannula during a procedure. Alternatively, the bulbous protrusion(s) may be attached to the cannula wall at the distal end of the cannula by various means such a medical grade adhesive or by welding.

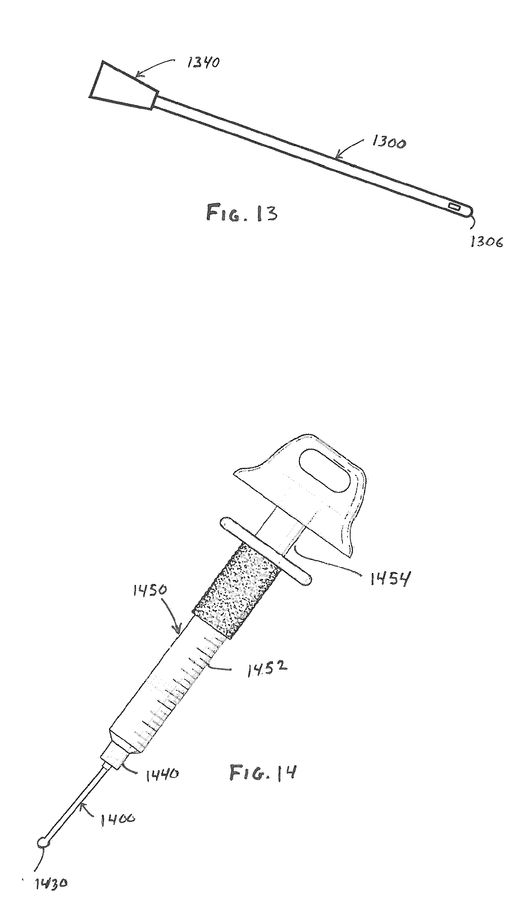

The cannulas disclosed herein may be operatively attached to an injection device for the injection of fluid(s) into the cannula, *i.e.,* into the fluid channel of the cannula. In this regard, **Fig. 13** illustrates a cannula **1300** having a blunt tip **1306** at a distal end **1308** of the cannula **1300.** An attachment device **1340** is provided at the proximal end **1312** of the cannula **1300** that is configured to attach the cannula **1300** to an injection device. For example, the attachment device **1340** may comprise a female Leur-Lok fitting that is configured to threadably attach to a male Leur-Lok fitting, such as on a syringe.

**Fig. 14** illustrates an assembly that includes a cannula that is operatively attached to a syringe. The syringe **1450** includes a barrel **1452** and a plunger **1454** that is operatively inserted into the barrel **1452.** A fluid may be contained within the barrel **1452** such that movement of the plunger **1454** causes movement of the fluid within the barrel **1452.** Examples of fluids that may be contained in the barrel **1452** include, but are not limited to, dermal fillers, autologous fat, medicaments, and the like.

A cannula **1400** is operatively attached to the syringe **1450** using an attachment device **1440** (*e.g*., a Leur-Lok) whereby the fluid channel of the cannula is in fluid communication with the barrel **1452.** The cannula may be configured in any manner disclosed herein, *e.g*., as illustrated in **Figs. 1-12** for example, the cannula **1400** may be substantially transparent such that fluids within the cannula **1400** (*e.g*., aspirated blood) may be visible along the length of the cannula **1400.** The cannula may also include a bulbous protrusion **1430** disposed at a distal end of the cannula **1400** such as for palpation of the cannula **1400.**

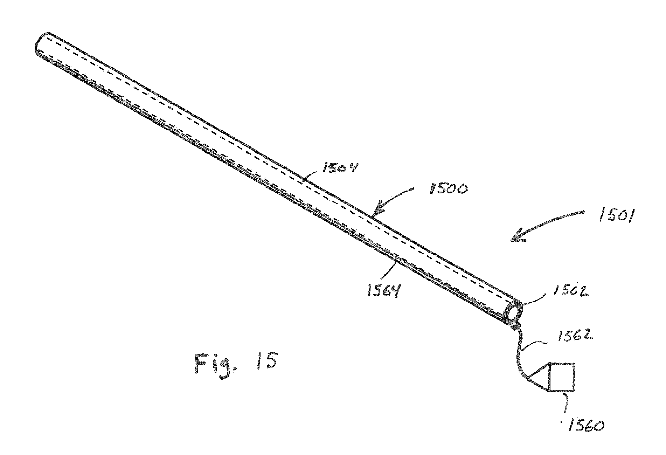

According to another embodiment, the cannula may be substantially transparent (*e.g*., translucent) and a light source may be operatively attached to the cannula to illuminate at least the cannula wall so that the location of the cannula in the deep/sub-dermal tissue of a patient may be more readily determined. **Fig. 15** illustrates a device **1501** that includes a cannula **1500** and a light source **1560** that is operatively coupled to the cannula **1500.** The light source **1560** may comprise, for example, a light-emitting diode (LED) or similar source of light. The light source **1560** may emit a wavelength of light that is approximately white, *e.g*., by mixing 3 primary colors. Alternatively, the light source **1560** may be monochromatic, such as by emitting yellow light such that different red and blue areas between the cannula wall **1504** and the skin surface are visible to an operator. The light source **1560** may also be characterized as a Class 1 light source. The light source may be operatively coupled to the cannula **1500** by an optical fiber **1562,** *e.g*., multi-mode optical fiber. As a result, light from the light source **1560** may be passed through the optical fiber **1562** and into the cannula wall **1502** to illuminate the cannula wall **1502.** In one characterization, an optical fiber **1564** (*e.g*., a second optical fiber) is integrally formed within the cannula wall **1504** and is accessible to the light source **1560** at an end of the cannula wall **1504.** In one characterization, the cannula wall **1502** is fabricated from a substantially transparent polymer, and in one particular characterization the cannula wall **1502** is formed from two or more layers of polymer having a different refractive index to facilitate the transmittance of light along the length of the cannula wall **1502.**

The present disclosure also includes methods for injecting an implant into the skin tissue of a mammalian patient. For example, the method may include forming an implantation puncture through the epidermis of a patient (*e.g*., using a sharp needle) removing the needle and inserting a blunt cannula into the implantation puncture. The cannula may be manipulated to a precise location and an implant material into the cannula. The implant material may comprise a dermal filler, autologous fat or a medicament, such as a long-term release medicament.

The method may also include aspirating blood after inserting the blunt tip of the cannula into the deep/sub-dermal tissue and observing, through the cannula wall and/or through a visualization port, an amount of aspirated blood in the fluid channel. The method may also include palpating to determine the position of a bulbous protrusion in the deep/sub-dermal tissue of the patient.

While various embodiments of the present invention have been described in detail, it is apparent that modifications and adaptations of those embodiments will occur to those skilled in the art. However, is to be expressly understood that such modifications and adaptations are within the spirit and scope of the present invention.

## Claims

1. A cannula configured for the injection of materials into the deep/sub-dermal tissue of a mammalian patient, comprising:
a longitudinally-extending cannula wall forming a circumferentially closed cylinder, the closed cylinder having an inner diameter defining a fluid channel along a length of the closed cylinder and having an outer diameter;
a blunt tip disposed at a distal end of the closed cylinder;
a proximal fluid port disposed at a proximal end of the closed cylinder and configured to provide fluid access to the fluid channel; and
at least a first distal fluid port disposed at a distal end of the closed cylinder and configured to provide fluid access to the fluid channel;
wherein the cannula wall is fabricated from a polymeric material.

2. The cannula recited in Claim 1, wherein the cannula wall is substantially transparent.

3. The cannula recited in any of the previous claims, wherein the polymeric material is an anisotropic polymer.

4. The cannula recited in any of the previous claims, wherein the polymeric material is an extruded polymeric material.

5. The cannula recited in any of the previous claims, wherein the polymeric material is selected from the group consisting of nylon, polypropylene, polyesters, polyamides, polyimides, polyurethanes, polyvinyl chloride (PVC), polyolefins and thermoplastic elastomers, polyacrylates, polycarbonates, polystyrenes, and modified or unmodified polyethylene terephthalates (PET).

6. The cannula recited in any of the previous claims, wherein the cannula wall has a thickness of at least about 0.0030 inches.

7. The cannula recited in any of the previous claims, wherein the cannula wall has a thickness of not greater than about 0.20 inches.

8. The cannula recited in any of the previous claims, wherein the fluid channel has a diameter of at least about 0.0010 inches and not greater than about 0.30 inches.

9. The cannula recited in any of the previous claims, wherein the cannula has a length of at least about 0.25 inches and not greater than about 6 inches.

10. The cannula recited in any of Claims 1-9, wherein the at least first distal fluid port is disposed through the blunt tip.

11. The cannula recited in any of Claims 1-9, wherein the at least first distal fluid port is disposed through the cannula wall.

12. The cannula recited in any of the previous claims, comprising at least a first visualization port disposed through the cannula wall nearer the proximal end of the cannula than the at least first distal fluid port.

13. The cannula recited in any of the previous claims, wherein the blunt tip comprises a bulbous protrusion having an outer diameter that is greater than the outer diameter of the closed cylinder.

14. The cannula recited in any of Claim 13, wherein the bulbous protrusion is substantially spherical.

15. The cannula recited in any of the previous claims, wherein at least a portion of the cannula wall is colored to facilitate visibility of the cannula wall when the cannula is at least partially inserted under the skin of a patient.

16. The cannula recited in any of the previous claims, comprising a light source operatively connected to the cannula and configured to illuminate the cannula wall when the cannula is at least partially inserted under the skin of a patient.
